# EUROPEAN PATENT SPECIFICATION

(11) **EP 4 132 937 B1**
(45) Date of publication and mention of the grant of the patent: **03.12.2025**
(21) Application number: 21723423.6
(22) Date of filing: 06.04.2021
(51) Int. Cl.: C07D 498/04, A01N 43/90

(54) **CO-CRYSTALS OF ANTIOXIDANTS AND ACTIVE INGREDIENTS AND USE OF ANTIOXIDANTS AS STABILIZER**
COKRISTALLE VON ANTIOXIDANTIEN UND WIRKSTOFFEN SOWIE VERWENDUNG VON ANTIOXIDANTIEN ALS STABILISATOR
CO-CRISTAUX D'ANTIOXYDANTS ET DE PRINCIPES ACTIFS ET UTILISATION D'ANTIOXYDANTS EN TANT QUE STABILISANTS

(30) Priority: 06.04.2020 US 202063005581 P; 26.07.2020 US 202063056659 P
(43) Date of publication of application: 15.02.2023
(73) Proprietor: Adama Agan Ltd., 7710201 Ashdod (IL)
(72) Inventor: GUBRIY, Vladimir, 7745510 Ashdod (IL); MOCATTA, David, 7044651 Gedera (IL); SILBERT, Gilad, 7917500 Kibbutz Dorot (IL)
(74) Representative: Modiano, Gabriella Diana
(86) International application number: PCT/IL2021/050392
(87) International publication number: WO 2021/205448

(56) References cited:
- WO-A1-2012/117362
- WO-A1-99/47525
- MICHEL MUEHLEBACH ET AL: "Aryldiones incorporating a [1,4,5]oxadiazepane ring. Part 2: Chemistry and biology of the cereal herbicide pinoxaden", PEST MANAGEMENT SCIENCE, vol. 67, no. 12, 8 June 2011 (2011-06-08), pages 1499 - 1521, XP055539325, ISSN: 1526-498X, DOI: 10.1002/ps.2204

## Description

### RELATED APPLICATION/S

This application claims the benefit of priority of U.S. Provisional Patent Application No. 63/005,581 filed on 6 April 2020 and U.S. Provisional Patent Application No. 63/056,659 filed on 26 July 2020.

### FIELD OF THE INVENTION

The present invention relates to the field of agrochemical formulations, more specifically to a cocrystal of an active ingredient, which is pinoxaden, and an antioxidant selected from the group consisting of butylated hydroxyanisole (BHA) and tert-butylhydroquinone (TBHQ). The invention is also directed to the use of said antioxidants for the stabilization of pinoxaden.

### BACKGROUND PRIOR ART

Herbicides are extensively used in the control of grasses and other weeds. Some herbicides display a 3-hydroxy-pyrazol skeleton containing a substituted phenyl group, for example, pinoxaden, which was disclosed as compound 1.008 in WO 99/47525 A1 (Novartis AG). It has been used together with different co-herbicides; see for example WO 01/17351 A1 (Syngenta Participations AG), which discloses mixtures of compounds of formula (I), for example, pinoxaden, with safeners having different formulas. Stabilizers and preservatives in general are mentioned as possible additives, but no more specific combinations are disclosed. Also, liquid formulations have been developed, for example as described in WO 2008/049618 A2 (Syngenta Participations AG), that required a built-in phosphate adjuvant. Pinoxaden and its herbicidal uses are further disclosed in "Pesticide Chemistry. Crop Protection, Public Health, Environmental Safety" ed. H. Ohkawa et al., 2007, Wiley, Weinheim, pp. 101-110.

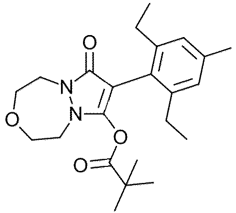

### Pinoxaden

Another representative compound of this structural family is pyraflufen-ethyl (not covered as such by the invention as claimed), disclosed as compound 262 in EP0361114A1.

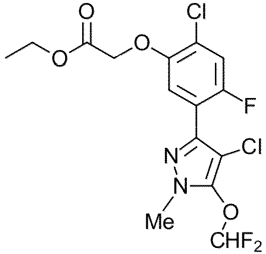

### Pyraflufen-ethyl

Pinoxaden and pyraflufen-ethyl (not covered as such by the invention as claimed) have however a problem of stability and their formulations, such as oil dispersions (OD), often result in the rapid degradation of the compound, typically due to hydrolization.

The same problem can be observed in sulfonylureas, such as mesosulfuron (not covered as such by the invention as claimed) which over time develop by-products having a negative impact on the quality of the product and limiting its useful shelf life.

WO0117351 discloses mixtures of pinoxaden with sulfonylureas. Stabilizers and preservatives in general are also mentioned as possible additives, but WO0117351 does not point to any specific combinations, and the examples do not disclose the addition of antioxidants.

WO0117352 discloses a mixture of a) a herbicidally effective amount of a compound of formula I (for example pinoxaden); b) a safener, for example, mefenpyr; and c) an oil. Stabilizers and preservatives in general are again mentioned as possible additives.

WO03028466 discloses mixtures of pinoxaden and mesosulfuron, optionally with mefenpyr. Stabilizers and preservatives in general are again mentioned as possible additives. US2009054236 discloses oil suspensions comprising a sulfonylurea and a safener, such as mefenpyr. Stabilizers, antioxidants and preservatives in general are mentioned as possible additives. Neither document points to combinations with antioxidants or to a possible stabilizing effect of antioxidants over pinoxaden or mesosulfuron.

WO07073933 is concerned with the problem of improving the stability of pinoxaden compositions. According to the document, pinoxaden is degraded over time due to hydrolysis or transesterification reactions. The document proposes to improve storage stability by the addition of an alcohol, which is said to prevent transesterification reactions. No mention is made of antioxidants or their possible stabilizing effect.

WO2018/108830 is also concerned with the stability of compositions, namely aqueous compositions, and provides a formulation with improved physical stability over a wide range of temperatures. The formulations disclosed comprise (i) an agrochemical; (ii) a non-ionic surfactant with a cloud point of 35-55°C and which is not component (iii) or (iv); (iii) an ethoxylated or propoxylated sorbitan ester; (iv) an alkyl polyglucoside; propylene glycol; glycerol; a glycol ether; or two or more of these compounds; and (v) water.

CN110402923A also discloses aqueous emulsions of pinoxaden. According to CN110402923A the stability is improved by adding an acrylic copolymer potassium salt as emulsifier and xanthan gum as thickener.

WO2014/060557 discloses pinoxaden compositions, typically in combination with florasulam, comprising a C₁-C₆ alkyl methacrylate polymer and a solvent comprising components (c1) and (c2), (c1) being a selection of alcoholic solvents, and (c2) being a heavy aromatic hydrocarbon solvent. In this way the use of tetrahydrofurfuryl alcohol is avoided. Antioxidants are generally mentioned as one of many additives that the formulation may contain. The document does not point to combinations with antioxidants or to a possible stabilizing effect of antioxidants.

WO2008/049618 discloses a composition comprising pinoxaden and a built-in adjuvant consisting of a tris-ester of phosphoric acid and/or a bis-ester of alkyl phosphonic acids with aliphatic or aromatic alcohols. Antioxidants are generally mentioned as one of many additives that the formulation may contain. The document does not point to combinations with antioxidants or to a possible stabilizing effect of antioxidants.

WO2012/117362 relates to stable pharmaceutical compositions of tetrahydrobiopterin and processes for producing such compositions. Particularly WO2012/117362 relates to stable pharmaceutical compositions comprising tetrahydrobiopterin and at least one stabilizing agent.

There is therefore in the art a need to provide improved formulations of herbicides such as (i) pinoxaden and improved formulations comprising (ii) pinoxaden and further herbicides.

### BRIEF DESCRIPTION OF THE SEVERAL VIEWS OF THE DRAWINGS

**Figure** 1: powder XRD diffractogram of a co-crystal of pinoxaden and BHA (top), XRD diffractogram of pinoxaden (middle) and XRD diffractogram of BHA (bottom).
**Figure** 2: powder XRD diffractogram of a co-crystal of pinoxaden and TBHQ (top), XRD diffractogram of pinoxaden (middle) and XRD diffractogram of TBHQ (bottom).

### SUMMARY OF THE INVENTION

In the search for a more stable formulation comprising pinoxaden, the inventors have tested many stabilizers without success, leading to unstable formulations. However, the addition of antioxidants selected from the group consisting of butylated hydroxyanisole (BHA) and *tert-*butylhydroquinone (TBHQ), typically used to stabilize oil carriers, surprisingly improved the stability of pinoxadenand of pinoxaden and sulfonylureas.

Thus, a first aspect of the present invention is the use of an antioxidant, selected from the group consisting of butylated hydroxyanisole (BHA) and tert-butylhydroquinone (TBHQ), to improve the stability of pinoxaden, stereoisomers, salts or solvates thereof, or of a mixture comprising pinoxaden and a sulfonylurea.

The inventors have found that pinoxaden and its derivatives form co-crystals with antioxidants selected from the group consisting of butylated hydroxyanisole (BHA) and *tert-*butylhydroquinone (TBHQ), a behavior that could not be anticipated.

It is thus a further aspect of the present invention a co-crystal comprising an antioxidant, selected from the group consisting of butylated hydroxyanisole (BHA) and tert-butylhydroquinone (TBHQ), and pinoxaden, stereoisomers, salts or solvates thereof.

A further aspect of the present invention is the use of a co-crystal comprising an antioxidant, selected from the group consisting of butylated hydroxyanisole (BHA) and *tert-*butylhydroquinone (TBHQ), and pinoxaden, stereoisomers, salts or solvates thereof, to improve the chemical stability of said compound with respect to the stability said compound would have in the absence of the antioxidant.

A further aspect of the present invention is the use of an antioxidant, selected from the group consisting of butylated hydroxyanisole (BHA) and tert-butylhydroquinone (TBHQ), to form a co-crystal comprising the antioxidant and pinoxaden, stereoisomers, salts or solvates thereof, so that the chemical stability of the pinoxaden, stereoisomers, salts or solvates thereof is improved.

As stated, the antioxidants encompassed by the invention are selected from the group consisting of butylated hydroxyanisole (BHA) and tert-butylhydroquinone (TBHQ.

A further aspect of the invention is a process for the preparation of a co-crystal according to the invention comprising mixing pinoxaden, stereoisomers, salts or solvates thereof with an antioxidant, selected from the group consisting of butylated hydroxyanisole (BHA) and *tert-*butylhydroquinone (TBHQ), in an organic solvent.

It is a further aspect of the invention a herbicidal formulation ("formulation according to the invention") comprising the co-crystal of the invention and optionally one or more agrochemical acceptable additives and/or carriers.

The co-crystals of the invention are formed *in situ* when mixing the pinoxaden, stereoisomers, salts or solvates thereof with the antioxidant, selected from the group consisting of butylated hydroxyanisole (BHA) and tert-butylhydroquinone (TBHQ).

However, the stabilizing action of the antioxidants, selected from the group consisting of butylated hydroxyanisole (BHA) and tert-butylhydroquinone (TBHQ), is not limited to the formation of co-crystals with pinoxaden, and the inventors have observed that they also have a surprising stabilizing effect on the sulfonylureas, when the latter are co-formulated along with the co-crystals of the invention.

Therefore, the formulation of the invention preferably comprises
(i) between 0.2 and 15 w/w% of pinoxaden, stereoisomers, salts or solvates thereof;
(ii) between 0.1 and 10 w/w % of a sulfonylurea;
(iii) between 0.01 and 10 w/w% of an antioxidant, selected from the group consisting of butylated hydroxyanisole (BHA) and tert-butylhydroquinone (TBHQ); and

optionally, any other agrochemical acceptable additives and/or solvents;
wherein said pinoxaden, stereoisomers, salts or solvates thereof and said sulfonylurea have improved stability when compared with the same composition without the antioxidant, selected from the group consisting of butylated hydroxyanisole (BHA) and tert-butylhydroquinone (TBHQ). The formulation of the invention preferably comprises (iv) between 1 and 15 w/w % of a safener.

A further aspect is the use of the formulation according to the invention for the control of undesired plants (also referred herein as "weed" or "undesired vegetation").

A further aspect is a method for the control of an undesired plant comprising contacting the formulation according to the invention with the locus of a crop.

A further aspect of the invention is a method for the preparation of a formulation according to the invention comprising contacting
(i) between 0.2 and 15 w/w% of pinoxaden, stereoisomers, salts or solvates thereof;
(ii) between 0.1 and 10 w/w % of a sulfonylurea; and
(iii) between 0.01 and 10 w/w% of an antioxidant, selected from the group consisting of butylated hydroxyanisole (BHA) and tert-butylhydroquinone (TBHQ), preferably between 1 and 10 w/w%, more preferably, between 2 and 10 w/w%.

The inventors have found that in the formulations comprising an antioxidant selected from the group consisting of butylated hydroxyanisole (BHA) and tert-butylhydroquinone (TBHQ), the chemical stability of pinoxaden, or of pinoxaden and sulfonylureas is significantly improved. Thus, a further aspect of the present invention is the use of an antioxidant, selected from the group consisting of butylated hydroxyanisole (BHA) and tert-butylhydroquinone (TBHQ), to prevent the degradation, to prevent the chemical degradation, or to improve the chemical stability of pinoxaden, stereoisomers, salts or solvates thereof, or of a mixture comprising pinoxaden and a sulfonylurea. The inventors have therefore found that antioxidants, selected from the group consisting of butylated hydroxyanisole (BHA) and tert-butylhydroquinone (TBHQ), can be used to better store and increase the shelf life of pinoxaden, stereoisomers, salts or solvates thereof, or of a mixture comprising pinoxaden and a sulfonyl urea.

Thus, a further aspect is a method for storing pinoxaden, stereoisomers, salts or solvates thereof, or a mixture comprising pinoxaden and sulfonyl urea, that comprises contacting said compounds with an antioxidant selected from the group consisting of butylated hydroxyanisole (BHA) and tert-butylhydroquinone (TBHQ). A further aspect is therefore the use of an antioxidant, selected from the group consisting of butylated hydroxyanisole (BHA) and tert-butylhydroquinone (TBHQ), for storing pinoxaden, stereoisomers, salts or solvates thereof, or a mixture comprising pinoxaden and a sulfonylurea.

Thus, a further aspect is a method of increasing the shelf life of pinoxaden, stereoisomers, salts or solvates thereof, or of a mixture comprising pinoxaden and sulfonylurea, that comprises contacting said compound(s) with an antioxidant selected from the group consisting of butylated hydroxyanisole (BHA) and tert-butylhydroquinone (TBHQ). Therefore, another aspect is the use of an antioxidant, selected from the group consisting of butylated hydroxyanisole (BHA) and *tert-*butylhydroquinone (TBHQ), for increasing the shelf life of pinoxaden, stereoisomers, salts or solvates thereof, or of a mixture comprising pinoxaden and a sulfonylurea.

### DETAILED DESCRIPTION OF THE INVENTION

### Definitions

In the present document the following terms are given the meaning below.

"Halogen" means in the present document as one of -F, -Cl, -Br or -I.

"Alkyl" means in the present document a straight or branched hydrocarbon chain radical consisting of carbon and hydrogen atoms, containing no unsaturation, having the number of carbon atoms indicated in each case, for example 1 to 16 carbon atoms, which is attached to the rest of the molecule by a single bond. In an embodiment of the invention the alkyl group comprises 1 to 8 carbon atoms. In a further embodiment it comprises 1 to 4 carbon atoms. Exemplary alkyl groups can be methyl, ethyl, n-propyl, i-propyl, n-butyl, t-butyl, or n-pentyl.

"Haloalkyl" means in the present document an alkyl group that is substituted with at least one of -F, -Cl, -Br or -I. The skilled person is aware of different substituents used frequently in organic chemistry, such as haloalkyl groups comprising 1, 2, 3, 4, 5, 6, 7 or 8 halogen substituents. Haloalkyl groups wherein all positions have been substituted with halogen atoms are also frequent (that is, perfluoro or perchloro substituents). Exemplary haloalkyl groups can be -CH₂F, -CH₂Cl, -CHF₂, -CF₃, -CCl₃, or -CF₂CF₃.

"Cyano" means in the present document -CN.

"Aryl" means in the present document an aromatic hydrocarbon radical having the number of carbon atoms indicated in each case, such as phenyl or naphthyl. The aryl radical may be optionally substituted by one, two or three groups selected from the group consisting of halogens, alkyl and haloalkyl groups.

"Heteroaryl" means in the present document a stable 3- to 15- membered cycloalkyl ring system attached to the rest of the molecule through a single bond, wherein one to five carbons atoms of the ring scaffold are replaced by heteroatoms selected from the group consisting of nitrogen, oxygen, and sulphur, and wherein at least one of the rings is aromatic. For the purposes of this invention, the heteroaryl may be a monocyclic, bicyclic or tricyclic ring system, which may include fused ring systems; and the nitrogen, carbon or sulphur atoms in the heteroaryl radical may be optionally oxidised; the nitrogen atom may be optionally quaternized. For example, an heteroaryl group can comprise a 4-to 8-membered ring with one or more heteroatoms, for example a 5-or 6-membered ring, wherein one, two or three carbons atoms of the ring scaffold are replaced by heteroatoms selected from the group consisting of nitrogen, oxygen, and sulphur, and wherein at least one of the rings is aromatic. Examples of heteroaryl groups include, but are not limited to pyridine, pyrazole, benzimidazole, benzothiazole, furan, isothiazole, imidazole, indole, purine, quinoline, thiadiazole.

The present document teaches that some of the compounds must be in an "herbicidal effective amount", which is understood as the amount of that compound that is effective to provide the desired effect when used in combination with the other components of the formulation. The amount that is "effective" will vary from application to application, depending on the particular crop to be treated or undesirable plant targeted. Additionally, what amount is considered effective may vary depending on the particular objective that is being pursued and on the balancing of certain outcomes, such as for example the effectiveness on the weed control versus the damage to crop. Thus, it is not always possible to specify an exact "effective amount", but in any given situation it may be determined by one of ordinary skill in the art using routine experimentation.

This is also true when determining the effective amount of a safener, a compound having antagonistic effects on the active herbicides present in the formulation, that is used to prevent damage to the crop.

The formulation of the invention is thus useful for the "control of undesired plants", which is given in the present document its usual meaning in the agrochemical industry, that is, amongst others, the capacity to kill, prevent growth or reproduction, or to diminish the health of unwanted plants in a given locus, by interfering in the unwanted plant's mechanisms, such as metabolism, photosynthesis and/or cell division.

"Crop" refers to any plant of industrial interest, including whole plants, plant organs (e.g. leaves, stems, twigs, roots, trunks, limbs, shoots, fruits etc.), plant cells, or plant seeds. This term also encompasses plant crops such as fruits. The term "plant" also includes the propagation material thereof, which may include all the generative parts of the plant such as seeds and vegetative plant material such as cuttings and tubers, which can be used for the multiplication of the plant. This includes seeds, tubers, spores, corms, bulbs, rhizomes, sprouts basal shoots, stolons, and buds and other parts of plants, including seedlings and young plants, which are to be transplanted after germination or after emergence from soil.

Locus are areas of land on which the plants already grow or in which the seeds of those plants are present or could be present, e.g. areas of land on which it is intended to grow cultivated plants or crops, or on which weeds are suspected to be or the grower wishes to prevent their growth.

Unless otherwise indicated, all weight percentages ("w/w %") are calculated with respect to the total weight of the formulation: 100x(weight of compound/total weight of formulation).

"Agrochemical acceptable" refers to any molecular entity and composition that is used in the formulation of the invention to improve any of its properties, such as any chemical, biological or physico-chemical properties approved for use in agrochemistry by a regulatory agency or a state government or other generally recognized pharmacopeia.

The invention also provides salts of the compounds herein defined. For instance, salts of compounds provided herein may be acid addition salts, base addition salts or metallic salts, and they can be synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

The term "solvate" according to this invention is to be understood as meaning any form of the compound which has another molecule (most likely a polar solvent) attached to it via non-covalent bonding. Examples of solvates include hydrates and alcoholates, e.g. methanolate.

An "stereoisomer" in the present patent application refers to compounds made up of the same atoms bonded by the same sequence of bonds but having different three-dimensional structures which are not interchangeable.

### Compounds of Formula (I)

The present invention refers to a multicomponent crystalline system that comprises an antioxidant, selected from the group consisting of butylated hydroxyanisole (BHA) and *tert-*butylhydroquinone (TBHQ), and a compound of formula (I), which is pinoxaden, stereoisomers, salts or solvates thereof. When combined, these species form a multicomponent crystal or a co-crystal, i.e. forming non-covalent intermolecular bonds, such as Van der Waals interactions. They can exist in various molar proportions, for example in a molar proportion of antioxidant, selected from the group consisting of butylated hydroxyanisole (BHA) and tert-butylhydroquinone (TBHQ ), to pinoxaden (BHA or TBHW:pinoxaden) comprised between 0.5 and 4, preferably between 0.5 and 2, preferably between 0.6 and 1.6. The pinoxaden, stereoisomers, salts or solvates thereof and the antioxidants, selected from the group consisting of butylated hydroxyanisole (BHA) and *tert-*butylhydroquinone (TBHQ), are present in the same solid phase as a homogeneous solid phase with a defined crystal structure, i.e. as co-crystals. That is, in the present invention a co-crystal is a mixed crystal which comprises, preferably consists of, two different components, in the case of the present invention, an antioxidant, selected from the group consisting of butylated hydroxyanisole (BHA) and tert-butylhydroquinone (TBHQ), and pinoxaden, stereoisomers, salts or solvates thereof. That is, both coexist within the same crystal.

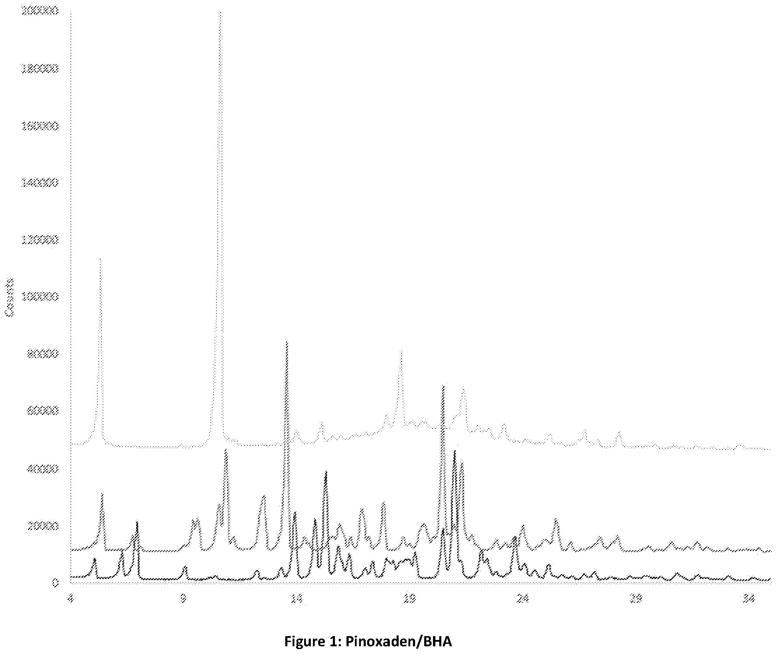

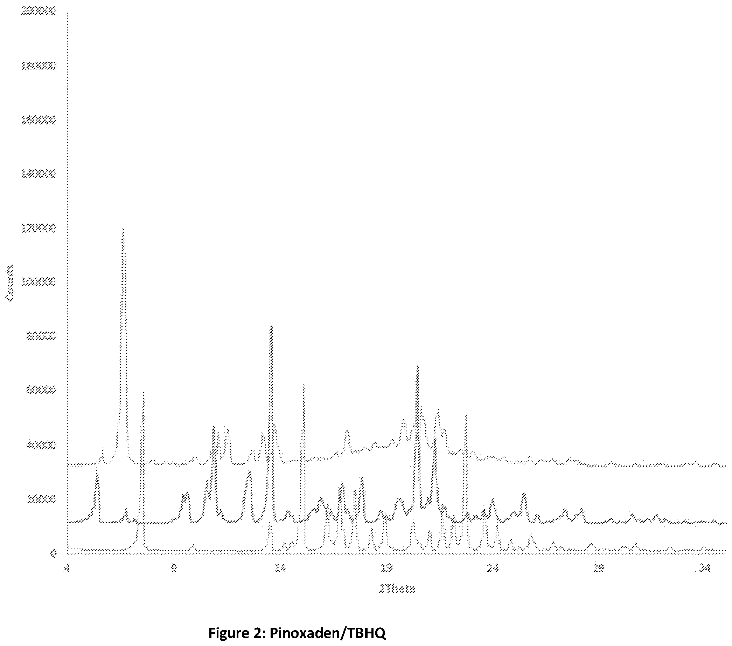

The co-crystals of the invention have clear crystalline powder XRD diffractograms that are distinguishable from those of their individual components (see Figures 1 and 2).

In the co-crystals of the invention the compound of formula (I) is pinoxaden or a stereoisomer, salt or solvate thereof.

Namely, in the present invention, the compound of formula (I) is pinoxaden, its stereoisomers, salts or solvates thereof.

All tautomers of pinoxaden are included in the scope of the present invention.

### Antioxidants

The antioxidants used in the present invention are selected from the group consisting of butylated hydroxyanisole (BHA) and tert-butylhydroquinone (TBHQ).

As mentioned above, the antioxidants of the invention, i.e. selected from the group consisting of butylated hydroxyanisole (BHA) and tert-butylhydroquinone (TBHQ), can form co-crystals with the pinoxaden, or stereoisomers, salts or solvates thereof. An exemplary co-crystal of the invention comprises pinoxaden, its stereoisomers, salts or solvates thereof and butylated hydroxyanisole (BHA). When mixed, both compounds form a co-crystal with a distinct structure wherein the XRD powder diffraction pattern shows at least one peak expressed as a 2θ angle selected from the group consisting of 5.32, 13.09, 15.08, 18.60 and 23.15. Preferably, the XRD powder diffraction pattern shows at least two, more preferably three peaks expressed as a 2θ angle selected from the group consisting of 5.32, 13.09, 15.08, 18.60 and 23.15. For example, the co-crystal formed by pinoxaden and BHA has an XRD powder diffraction pattern showing the following peaks expressed as a 2θ angle: 5.32, 13.09, 15.08, 18.60 and 23.15. A complete powder XRD diffractogram is show in Figure 1, which is also an embodiment of the invention.

An exemplary co-crystal of the invention comprises pinoxaden, its stereoisomers, salts or solvates thereof and tert-butylhydroquinone (TBHQ). When mixed, both compounds form a co-crystal with a distinct structure wherein the XRD powder diffraction pattern shows at least one peak expressed as a 2θ angle selected from the group consisting of 5.64, 6.64, 7.95, 11.55, 13.18, 22.47, 23.11 and 24.55. Preferably, the XRD powder diffraction pattern shows at least two, more preferably three, more preferably four, even more preferably five peaks expressed as a 2θ angle selected from the group consisting of 5.64, 6.64, 7.95, 11.55, 13.18, 22.47, 23.11 and 24.55. For example, the co-crystal formed by pinoxaden and TBHQ has a XRD powder diffraction pattern showing the following peaks expressed as a 2θ angle: 5.64, 6.64, 7.95, 11.55, 13.18, 22.47, 23.11 and 24.55. A complete powder XRD diffractogram is show in Figure 2, which is also an embodiment of the invention.

The cocrystals of the invention can be prepared by dissolving the antioxidant, selected from the group consisting of butylated hydroxyanisole (BHA) and tert-butylhydroquinone (TBHQ), and the pinoxaden, stereoisomers, salts or solvates thereof in a suitable carrier and allowing the crystals to be formed. Suitable carriers are oils, for example, canola oil. The stirring speed is not critical for the formation of the cocrystals and they can be formed, for example, at stirring comprised between 0 and 3000 rpm. For example, the crystals can be obtained by first dissolving in an oil (e.g. canola oil) at room temperature (15-25°C) the antioxidant, selected from the group consisting of butylated hydroxyanisole (BHA) and tert-butylhydroquinone (TBHQ). Then, the pinoxaden can be dissolved at the same temperature. The mixture can be allowed to sit or stir, for example, for 0.1 hours to 15 days (typically 0.1-48 hours), and the crystals formed filtered or decanted. The filtrate can be alternatively washed with cold hexane.

### Sulfonylureas

The formulations, uses and methods of the invention comprise pinoxaden, its stereoisomers, salts or solvates thereof, or a mixture of pinoxaden and sulfonylurea.

Sulfonylureas are a well know family of ALS inhibitors with numerous examples in the pesticide industry. For example, Triasulfuron, tribenuron-methyl, iodosuifuron-methyl (as the sodium salt), mesosulfuron- methyl, and pyroxsulam are disclosed in "The Pesticide Manual, ed. C.D.S. Tomlin, 15th edition, 2009, British Crop Production Council, UK, see entry 494 "iodosulfuron-methyl- sodium" (pp. 658-660), entry 550 "mesosulfuron-methyl" (pp. 733-734), entry 753 "pyroxsulam" (pp. 1001-1002), entry 868 "triasulfuron" (pp. 1150-1151), and entry 873 "tribenuron-methyl" (pp. 1156-1158).

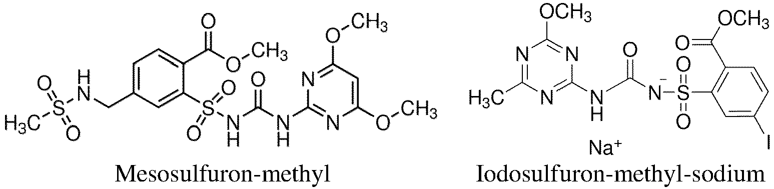

Preferably, the sulfonylurea herbicide is one selected from the group consisting of amidosulfuron, azimsulfuron, bensulfuron, chlorimuron, chlorsulfuron, cinosulfuron, cyclosulfamuron, ethametsulfuron, ethoxysulfuron, flazasulfuron, flucarbazone, flupyrsulfuron, foramsulfuron, halosulfuron, imazosulfuron, iodosulfuron, mesosulfuron, nicosulfuron, oxasulfuron, primisulfuron, propoxycarbazone, prosulfuron, pyrazosulfuron, rimsulfuron, sulfometuron, sulfosulfuron, thiencarbazone, thifensulfuron, triasulfuron, tribenuron, trifloxysulfuron, triflusulfuron, tritosulfuron and alkyl ethers or esters thereof, as well as salts thereof.

For example, the sulfonylurea herbicide can be one of compound of formula (II), its isomers, stereoisomers, salts and solvates thereof 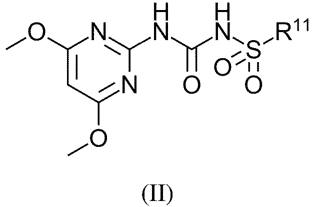 wherein
R¹¹ is phenyl or heterocyclyl, either optionally substituted by one or two groups selected from the group consisting of halogen, cyano, C₁-C₆-alkyl, C₁-C₆-haloalkyl, -(C=O)-R⁷, - (C=O)-O-R⁷, -(C=O)-N(R⁷)(R⁸), -SO₂-R⁷, and C₁-C₆-alkyl-N(H)-SO₂-R⁷, wherein R⁷ is selected from the group consisting of C₁-C₆-alkyl, C₁-C₆-alkyl-C₆-C₁₀-aryl, and C₆-C₁₀-aryl-C₁-C₆-alkyl; and R⁸ is selected from the group consisting of hydrogen, C₁-C₆-alkyl, C₁-C₆-alkyl-C₆-C₁₀-aryl, and C₆-C₁₀-aryl-C₁-C₆-alkyl.

It is preferred that in the compounds of formula (II) R¹¹ is phenyl or pyridine, either optionally substituted by one or two groups selected from the group consisting of halogen, cyano, C₁-C₆-alkyl, -(C=O)-O-R⁷, and C₁-C₃-alkyl-N(H)-SO₂-R⁷, wherein R⁷ is selected from the group consisting of C₁-C₆-alkyl; and R⁸ is selected from the group consisting of hydrogen, and C₁-C₆-alkyl.

More preferably, the compound of formula (II) is mesosulfuron or a mesosulfuron ester, for example mesosulfuron-methyl.

The sulfonylurea can also be a compound of formula (V), its isomers, stereoisomers, salts and solvates thereof 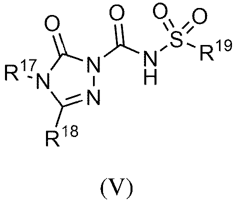 wherein
R¹⁷ is selected from the group consisting of hydrogen, C₁-C₆-alkyl, C₁-C₆-alkyl-C₆-C₁₀-aryl, and C₆-C₁₀-aryl-C₁-C₆-alkyl;
R¹⁸ is selected from the group consisting of hydrogen, C₁-C₆-alkyl, C₁-C₆-haloalkyl, OR²⁰, -(C=O)-O-R²⁰, wherein R²⁰ is selected from the group consisting of C₁-C₆-alkyl, C₁-C₆-alkyl-C₆-C₁₀-aryl, and C₆-C₁₀-aryl-C₁-C₆-alkyl; and
R¹⁹ is phenyl or heterocyclyl, either optionally substituted by one or two groups selected from the group consisting of halogen, cyano, C₁-C₆-alkyl, C₁-C₆-haloalkyl, -(C=O)-R ⁷, - (C=O)-O-R⁷, -(C=O)-N(R⁷)(R⁸), -SO₂-R⁷, and C₁-C₆-alkyl-N(H)-SO₂-R⁷, wherein R⁷ is selected from the group consisting of C₁-C₆-alkyl, C₁-C₆-alkyl-C₆-C₁₀-aryl, and C₆-C₁₀-aryl-C₁-C₆-alkyl; and R⁸ is selected from the group consisting of hydrogen, C₁-C₆-alkyl, C₁-C₆-alkyl-C₆-C₁₀-aryl, and C₆-C₁₀-aryl-C₁-C₆-alkyl.

For example, a compound of formula (V) can be thiencarbazone-methyl, flucarbazone-sodium or propoxycarbazone-sodium.

The fact that the antioxidants, selected from the group consisting of butylated hydroxyanisole (BHA) and tert-butylhydroquinone (TBHQ), are capable of stabilizing both, the pinoxaden and sulfonylureas, such as the compounds of formula (II) (e.g. mesosulfuron) or the compounds of formula (V) (e.g. thiencarbazone), was even more surprising since both are structurally distinct.

Combinations of pinoxaden and structural derivatives thereof together with sulfonylureas are known in the art. WO 2011/107741 A1 discloses herbicidal compositions comprising a mixture of (a) an ALS inhibitor in the form of an aluminum salt and (b) pinoxaden. The examples include the simultaneous treatment with pinoxaden commercial formulations (including a safener) and with dicamba salts, trisulfuron, tribenuron-methyl commercial formulations. However, it does not disclose the addition of antioxidants to the formulation, not to mention that they could improve the chemical stability of a sulfonylurea, of pinoxaden, stereoisomers, salts or solvates thereof, or of a mixture comprising both.

Thus, the invention preferably refers to the use of an antioxidant, selected from the group consisting of butylated hydroxyanisole (BHA) and tert-butylhydroquinone (TBHQ), to improve the stability of a mixture comprising (i) pinoxaden; and (ii) a sufonylurea, preferably a mixture comprising (i) pinoxaden; and (ii) mesosulfuron or mesosulfuron-methyl.

### Safeners

When using herbicides, the crops may also suffer damage, depending on, for example, the amount of herbicide used and the method of application, the nature of the soil or the climatic conditions, for example hours of daylight, temperature and amount of rainfall. Various safeners (previously known as "antidotes") are known to antagonize the harmful effect of the herbicide on crops and protect them without appreciably impairing the herbicidal action on the undesirable plants to be controlled.

These substances are known in the agricultural industry and are taught in different reviews, for example, Délye C, et al (2017) "Herbicide Safeners Decrease Sensitivity to Herbicides Inhibiting Acetolactate-Synthase and Likely Activate Non-Target-Site-Based Resistance Pathways in the Major Grass Weed Lolium sp. (Rye-Grass)" Front. Plant Sci. 8:1310, doi: 10.3389/fpls.2017.01310; Davies, J. "Herbicide safeners - commercial products and tools for agrochemical research", Pesticide Outlook, February 2001, p. 10-15 10.1039/b100799h; Jablonkai, I. "Herbicide Safeners: Effective Tools to Improve Herbicide Selectivity" Intech open Science, www.dx.doi.org/10.5772/55168; Rosinger, C. "Sektion 6: Developments in herbicides - Herbicide Safeners: an overview", 26th German Conference on weed Biology an Weed Control, March 11-13, 2014, Braunschweig, Germany.

Exemplary safeners that can be used in the present invention are anhydrides (e.g. 1,8-naphthalic anhydride), dihydropyrazole-dicarboxylates (e.g. mefenpyr), dihydroisoxazole-carboxylates (e.g. isoxadifen), thiazole carboxylic acids (e.g. flurazole), phenyl-pyrimidines (e.g. fenclorim), 1,2,4-Triazole-carboxylates (e.g. fenchlorazole), dichloroacetamides (e.g. dichlormid), Ureas (e.g. dymron), arylsulfonyl-benzamides (e.g. cyprosulfamide), oxime ethers (e.g. cyometrinil), 8-Quinolinoxy-carboxylic esters (e.g. cloquintocet), dichloromethylketals (e.g. MG-91), or piperidine-1-carbothioates (e.g. Dimepiperate).

For example, known safeners that can be used in the present invention are oxabetrinil, 1,8-naphthalic anhydride, metcamifen, mephenate, mefenpyr, jiecaoxi, jiecaowan, isoxadifen, furilazole, fluxofenim, flurazole, fenclorim, fenchlorazole, dietholate, dicyclonon, dichlormid, cyprosulfamide, cyometrinil, cloquintocet, AD-67, BPCMS, MG-91, Dymron, benoxacor or salts, solvates or esters thereof.

A preferred group of safeners in the present invention are the dihydropyrazole-dicarboxylates or the 8-Quinolinoxy-carboxylic esters. More preferably, the formulation of the invention comprises a safener selected from the group consisting of cloquintocet or a salt thereof, preferably an alkali, alkaline earth, sulfonium or ammonium cation of cloquintocet, cloquintocet-mexyl, mefenpyr or a salt thereof, an alkali, alkaline earth, sulfonium or ammonium cation of mefenpyr, mefenpyr-diethyl and mixtures thereof, preferably mefenpyr-diethyl.

A safener in accordance with the invention may, depending on the intended purpose, be used to pre-treat a seed material of the cultivated plant (dressing the seed or the seedlings) or may be incorporated into the soil before or after sowing. It may, however, also be applied, alone or together with one or more herbicides, after the emergence of the plants. The treatment of the plants or the seeds with the safener can therefore, in principle, be effected independently of the time at which the herbicide is applied. The treatment of the locus can, however, also be carried out by applying the safener simultaneously with the pinoxaden, stereoisomers, salts or solvates thereof, or of a mixture comprising pinoxaden and a sulfonylurea (for example in the form of a tank mixture). In the present invention it is preferred that the safener is part of the same formulation as the pinoxaden, stereoisomers, salts or solvates thereof, or the mixture comprising the pinoxaden and the sulfonylurea, and is preferably used as post-emergence control. The rate of application of the safener in relation to the herbicide or herbicides depends largely on the method of application. For example, the molar ratio of compound of herbicides:safener is generally from 100:1 to 1:10, preferably from 20:1 to 1:2, for example 3:1 to 1:1.5, wherein the herbicide can be the pinoxaden, stereoisomers, salts or solvates thereof, or a mixture comprising the pinoxaden and a sulfonylurea.

In terms of the amounts of safener to be applied, the present invention can use customary amounts, for example, from 0.001 to 1.0 kg of safener/ha, preferably from 0.001 to 0.25 kg of safener/ha, are generally applied.

### Co-herbicides

The formulations, uses and methods of the invention may comprise at least one co-herbicide different from pinoxaden and from the combination of pinoxaden and sulfonylureas, for example one selected from the group consisting of other ALS inhibitors such as sulphonyl-carbonyl-triazolinones, imidazolinones, triazolopyrimidines or pyrimidinyl (thio) benzoates; inhibitors of the photosynthesis at PS II such as nitriles, phenyl-pyridazines, benzothiadiazinones, triazines, triazolinones or triazinones; PS-I-electron diversion such as bipyridyliums; protoporphyrinogen oxidase inhibitors, such as diphenyl ethers, thiadiazoles, pyrmidinediones, N-phenyl-phthalimides, oxazolidinediones, tiazolinones, oxadiazoles or phenypyrazoles; PDS inhibitors; 4-HPPD inhibitors; DOXP synthase inhibitors and other pigment synthesis inhibitors; EPSP synthase inhibitors; glutamine synthase inhibitors; DHP inhibitors; microtubule assembly inhibitors; microtubule organization inhibitors; VLCFA inhibitors; inhibitors of the synthesis of cellulose; membrane disruptors (uncouplers); non-ACCase inhibitors of lipid synthesis; synthetic auxins; or auxin transport inhibitors.

Herbicides that can be used as co-herbicides in the present invention can be for example imazapyr, pyrithiobac-sodium, pyriminobac, bispyribac-sodium, atrazin, butracil, simazin, simethryne, terbutryne, terbuthylazine, trimexyflam, isoproturon, chlortoluron, diuron, dymron, fluometuron, linuron, methabenzthiazuron, glyphosate, sulfosate, glufosinate, nitrofen, bifenox, acifluorfen, lactofen, oxyfluorfen, ethoxyfen, fluoroglycofen, fomesafen, halosafen, azafenidin, benzfendizone, butafenacil, carfentrazone-ethyl, cinidon-ethyl, flumichlorac-pentyl, flumioxazin, fluthiacet-methyl, oxadiargyl, oxadiazon, pentoxazon, sulfentrazone, fluazolate, pyraflufen-ethyl, alachlor, acetochlor, butachlor, dimethachlor, dimethenamid, S-dimethenamid, metazachlor, metolachlor, S-metolachlor, pretilachlor, propachlor, propisochlor, thenylchlor, pethoamid, 2,4-D, fluroxypyr, MCPA, MCPP, MCPB, trichlorpyr, mecropop-P, hexazinon, metamitron, metribuzin, oryzalin, pendimethalin, trifluralin, chloridazon, norflurazon, chlorpropham, desmedipham, phenmedipham, propham, mefenacet, fluthiacet, butylate, cycloate, diallate, EPTC, esprocarb, molinate, prosulfocarb, thiobencarb, triallate, fentrazamide, cafenstrole, dicamba, picloram, diflufenican, propanil, bromoxynil, dichlobenil, ioxynil, sulcotrione, mesotrione, isoxaflutole, isoxachlortole, flucarbazone, propoxycarbazone (or its sodium salt), foramsulfuron, penoxsulam, trifloxysulfuron (or its sodium salt), pyriftalid, trifloxysulfuron (or its sodium salt), pyriftalid, flufenpyr-ethyl, profluazol, pyraclonil, benfluamid, picolinafen, amicarbazone, flufenpyr-ethyl, profluazol, pyraclonil, benfluamid, picolinafen, amicarbazone, chlorasuiam, diclosulam, florasulam, flumetsulam, metosulam, amitrol, benfuresate, bentazone, cinmethylin, clomazone, chlopyralid, difenzoquat, dithiopyr, ethofumesate, flurochloridone, indanofane, isoxaben, oxaziclomefone, pyridate, pyridafol, quinchlorac, quinmerac, tridiphane and flamprop.

### Formulations

The pinoxaden can be added in different amounts, typically between 0.1 w/w % and 70 w/w %, for example, between 0.1 and 50 w/w % or between 0.5 and 10 w/w % or between 1 and 8 w/w %. The loading of the pinoxaden can depend on the type of formulation used. For example, in the present application a suspension of the pinoxaden is preferred, typically an oil-in-water emulsion (EW), a microemulsion (ME), an oil-based suspension concentrate or oil concentrate (OD), the formulation preferably being an OD comprising between 0.1 and 15 w/w % of pinoxaden stereoisomers, salts or solvates thereof.

The concentration of the antioxidant, selected from the group consisting of butylated hydroxyanisole (BHA) and tert-butylhydroquinone (TBHQ), in the mixture or formulation can for example be from 0.01 to 10 w/w %, for example from 0.02 to 10 w/w %, for example, from 0.05 to 8 w/w %. Although antioxidants are sometimes added in small amounts to prevent degradation of oil carriers, the inventors have realized that higher amounts of antioxidant, selected from the group consisting of butylated hydroxyanisole (BHA) and tert-butylhydroquinone (TBHQ), improve the chemical stability of pinoxaden, stereoisomers, salts or solvates thereof or of a mixture comprising pinoxaden and a sulfonylurea. Thus, the concentration of the antioxidant, selected from the group consisting of butylated hydroxyanisole (BHA) and tert-butylhydroquinone (TBHQ), in the mixture of formulation can be from 0.5 to 7 w/w %, or from 1 to 7 w/w %, or 2 to 6 w/w%, or from 1 to 5 w/w %. Typically, the concentration of the antioxidant, selected from the group consisting of butylated hydroxyanisole (BHA) and tert-butylhydroquinone (TBHQ), can be for 2 to 4.8 w/w % or from 3 to 4.5 w/w %.

The sulfonylurea can be present, together with the pinoxaden, in a wide range of concentrations, typically, in an amount from 0.01 to 95 w/w %, typically from 0.1 to 50 w/w %, for example, from 0.2 to 5 w/w %, or from 0.5 to 4 w/w %, or from 0.5 to 3 w/w %. Sufonylureas can even be in lower amounts, for example, between 0.01 and 0.5 w/w %. The formulations of the invention may comprise from 0.01 to 95 w/w %, typically from 0.1 to 55 w/w %, of total active ingredients, for example, from 0.5 to 20 w/w %, or from 1 to 15 w/w % of total active ingredients, considering total amount of active ingredients as the sum of pinoxaden, stereoisomers, salts or solvates thereof, sulfonylureas, other co-herbicides and any safeners.

The concentration of the safener in the application can for example be from 0.01 to 95 w/w %, typically from 0.1 to 50 w/w %, for example, from 0.5 to 10 w/w %, from 0.5 to 7 w/w %, or from 1 to 5 w/w %.

The formulations of the invention may also comprise further additives commonly used in the field of agrochemistry, such as wetting agents, adjuvants, adhesives, neutralizers, antifoams, for example silicone oil, sequestrates, fertilizers, biocides, viscosity regulators, binders, tackifiers, thickeners, buffers or anti-freeze agents, or surfactants, amongst others. The formulations are prepared in a known manner, for example by intimately mixing and/or grinding the active ingredients or optionally with other solid components, with the oil carrier.

Said additives are known in the art and the skilled person can find numerous examples in the literature. For examples, McCutcheon's, "Detergents and Emulsifiers Annual", MC Publ. Corp., Ridgewood NJ.; or Sisley and Wood, "Encyclopedia of Surface Active Agents", Chem. Publ. Co. Inc., N.Y. 1964; provide detailed examples of surfactants that can be useful. Surfactants can be anionic surfactants of the dodecylbenzylsulfonate type (alkyl benzene sulfonate salts such as Nansa EVM 70/2E)), especially the calcium salts thereof, and also non-ionic surfactants of the fatty alcohol ethoxylate type. Examples of commercially available surfactants are the Genapol types (Clariant AG, Muttenz, Switzerland). Typical surfactants can also be selected from the group consisting of polyacrylate graft copolymers (e.g. Dispersogen PSL 100), phosphated alcohol ethoxylates (e.g. Hostaphat 1306) and star structure polymers (e.g. Atlox 4916). The concentration of the surface-active substances is generally from 1 to 30 w/w %, preferably between 2 and 10 w/w %.

The antifoams or defoamers useful in the formulations of the invention can be selected from the group consisting of silicon oil, polyethylene glycol polypropylene glycol copolymers and alkyl polyacrylates.

The thickeners useful in the formulations of the invention can be selected from the group consisting of organically-derived hectorite clay (e.g. Bentone 38), polyester block co-polymer (e.g. Atlox Rheostrux 100), organically-derived silica (e.g. Aerosil R816). The formulations, methods and uses of the present invention will typically use suitable carriers that can be aqueous or organic solvents, depending on the type of formulation being used.

The skilled person has different types of formulations available. Liquid formulations are preferred in the formulations, methods and uses according to the present invention, for example, solutions for seed treatment (LS), oil-miscible liquids (OL), soluble concentrates (SL), ultra-low-volume (ULV) liquids, dispersable concentrates (DC), emulsifiable concentrates (EC), emulsions for seed treatment (ES), oil-in-water emulsions (EW), microemulsions (ME), aqueous capsule suspensions (CS), suspension concentrates for seed treatment (FS), oil dispersion (OD), aqueous suspension concentrates (SC), aqueous suspo-emulsions (SE), mixed aqueous suspension concentrate and aqueous capsule suspension (ZC), mixed oil-in-water emulsions and aqueous capsule suspension (ZW), or mixed aqueous suspo-emulsions and aqueous capsule suspension (ZE).

More preferable types of formulations are emulsifiable concentrates (EC), and oil dispersions (OD), a dispersable concentrates (DC) or microemulsions (ME), oil dispersions (OD) being the most preferred. The oil carrier used in OD formulation can be a mineral oil or a vegetable oil such as, for example, rapeseed oil, olive oil or sunflower oil, an emulsified vegetable oil, or an alkyl ester of an oil of vegetable origin such as, for example, the methyl or ethyl ester derivatives, or an oil of animal origin such as fish oil or beef tallow. Mineral oils can be aromatic or nonaromatic, preferably paraffins, such as isoparaffins. Exemplary isoparaffins comprise C₈-C₂₅, for example C₁₀-C₂₀-isoalkanes, i.e. alkanes comprising mostly linear molecules with a methyl group in one of its penultimate carbon atoms to form a -C(H)(CH₃)₂ residue. It is for example preferred that the oil carrier is an isoparaffinic oil comprising at least 20 w/w % of C₁₄-C₁₉-isoalkanes, with respect to the total weight of the oil, and less than 2 w/w % of aromatic compounds, with respect to the total weight of the oil. The oil carrier in the OD formulation can be used in amounts above 10 w/w %, more preferably above 20 w/w %, or above 40 w/w %, or above 50 w/w %, or above 70 w/w %, or above 80 w/w %, for example, between 75 w/w % and 99 w/w %, or between 80 and 95 w/w %.

### Methods

The formulations, methods and uses of the invention are compatible with all methods of application that are customary in agriculture, for example pre-emergence application, post-emergence application and seed dressing.

Pinoxaden, stereoisomers , salts or solvates thereof are known to be suitable for use on cereals, preferably non-oat cereals such as wheat, and on barley, rye and/or triticale, especially wheat and/or barley (i.e. is selective for non-oat cereals), and is typically applied post-emergence for control of grassy weeds such as Alopecurus, Apera, Avena, Lolium, Phalaris or Setaria species, e.g. at application rates of from 30 to 60 g/ha (ha = hectare).

The invention thus relates also to a method for the control of an undesired plant comprising contacting the formulation according to the invention with the locus of said undesired plant. Herbicides are frequently sold in concentrated form which can then be diluted in aqueous composition before application. The formulation according to the invention can thus be dispersed in an aqueous solution, optionally in the presence of one or more adjuvants, and then applied to the locus of the plant.

Crops are to be understood as also including those that have been made tolerant to herbicides or classes of herbicides by means of conventional breeding or genetic engineering methods.

The undesired plants to be controlled may include one or more of Alfalfa (Medicago sativa), Amsinckia (Amsinckia spp.), Capeweed (Arctotheca calendula), Charlock (wild mustard, Sinapis arvensis), Chickweed (Stellaria media), Corn gromwell (sheepweed, white ironweed) (Buglossoides arvensis), Common couchgrass (Agropyron repens), Corn mint (Mentha arvensis), Crassula (Crassula spp.), Creeping thistle (Cirsium arvense), False bindweed (Calystegia sepium), False mayweed (Tripleurospermum maritimum), Field bindweed (Convolvulus arvensis), Fool's parsley (Aethusa cynapium), Gallant soldier (Galinsoga parviflora), Henbit deadnettle (Lamium amplexicaule), Hedge mustard (Sisymbrium officinale), Hyssop loosestrife (Lythrum hyssopifolia), Indian hedge mustard (Sisymbrium orientale), Italian rye-grass (Lolium multiflorum), Marshmallow (Malva parviflora), Sticky mouse-eared chickweed (Cerastium glomeratum), Night-scented stock (Matthiola longipetala), Paterson's curse (Salvation Jane) (Echium plantagineum), Pheasant's eye (Adonis microcarpa), Pineapple weed (Matricaria discoidea), Poinsettia (Euphorbia pulcherrima), Prickly lettuce (Lactuca serriola), Purple deadnettle (Lamium purpureum), Rough poppy (Papaver hybridum), Shepherd's purse (Capsella bursa- pastoris), Skeletonweed (Chondrilla juncea), Sorrel (Rumex acetosella), Ivy-leaved Speedwell (Veronica hederifolia), Dwarf nettle (Stinging nettle) (Urtica urens), Toad rush (Juncus bufonius), Turnipweed (Rapistrum rugosum), Wild radish (Raphanus raphanistrum), Wild turnip (Brassica tournefortii), Knotgrass (Polygonum aviculare), Annual mercury (Mercurialis annua), False castor oil (thomapples) (Datura ferox), Ball mustard (Neslia paniculata), Barnyard grass (Echinochloa crusgalli), Beta vulgaris, Black Bindweed (Fallopia convolvulus), Blackgrass (Alopecurus myosuroides), Black pigweed (Trianthema portulacastrum), Buchan weed (Hirschfeldia incana), Bull's head (Tribulus terrestris), Buttonweed (Abutilon theophrasti), Catsear (Hypochaeris radicata), Cleavers (Galium aparine), Common bugloss (Anthusa arvensis), Common borage (Boragio officinales), Common cotula (Cotula australis), Common groundsel (Senecio vulgaris), Common hempnettle (Galeopsis tetrahit), Common nettle (Urtica dioica), Corn spurry (Spergula arvensis), Creeping speedwell (Veronica filiformis), Crown beard (dogweed) (Verbesina encelioides), Crowsfoot grass (Eleusine indica), Dock (Rumex spp.), Doublegee (Emex australis), Fat hen (Atriplex prostrata), Field madder (Sherardia arvensis), Field Pansy (Viola arvensis), Field oennycress (Thlaspi arvense), Fog grass (Holcus lanatus), Fumitory (Fumaria spp.), Fumaria officinalis, Geranium (Geranium spp.), Geranium dissectum, Great brome (Bromus diandrus), Greater plantain (Plantago major), Green summer grass (Digitaria ciliaris), Hare's ear (treacle mustard) (Conringia orientalis), Heliotrope (Heliotr opium spp.), Hempnettle (Galeopsis sp.), Horehound (Marrubium vulgare), Indian hedge mustard (Sisymbrium orientale), Lesser swinecress (Coronopus didymus), Matricaria spp., Scented mayweed (Matricaria recutita), Scentless mayweed (Matricaria inodora), Mountain sorrel (purple calandrinia) (Calandrinia ciliata), Mustard, Nettle, Urtica spp., Nightshade (Solanum nigrum), Pale persicaria (Persicaria lapathifolia), Perennial sowthistle (Sonchus arvensis), Persian speedwell (Veronica persica), Chenopodium sp., Pigweed (Chenopodium albu ), Powell's amaranth (Amaranthus powellii), Red hempnettle (Galeopsis intermedia), Redshank (Persicaria maculosa), Redroot pigweed (Amaranthus retroflexus), Scarlet pimpernel (Anagallis arvensis), Scrub nettle (stinging nettle) (Urtica incisa), Annual sowthistle (Sonchus oleraceus), Spiny emex (Three cornered jack) (Emex australis), Spreading orache (Atriplex patula), Stagger weed (Stachys arvensis), Storksbill (Erodium cicutarium), Summer grass, Sun spurge (Euphorbia helioscopia), Toad rush (Juncus bufonius), Black bindweed (Polygonum convolvulus), Polygonum spp., Wild Pansy (Viola tricolor), Winter grass (annual meadow grass) (Poa Annua), Wild oat (Avena fatua), Winter rape (Brassica napus) and Yellow burrweed (Amsinckia calycina).

Preferably, the undesirable weeds to be controlled are monocotyledonous weeds, for example the monocotyledonous weeds Avena, Agrostis, Phalaris, Lolium, Bromus, Alopecurus, Setaria, Digitaria, Brachiaria, Echinochloa, Panicum, Sorghum hal./bic., Rottboellia, Cyperus, Brachiaria, Echinochloa, Scirpus, Monochoria and Sagittaria and the dicotyledonous weeds Sinapis, Chenopodium, Stellaria, Galium, Viola, Veronica, Matricaria, Papaver, Solanum, Abutilon, Sida, Xanthium, Amaranthus, Ipomoea and Chrysanthemum.

The rate of application of the pinoxaden is generally from 0.001 to 2 kg/ha, but preferably from 0.005 to 1 kg/ha.

The nature of the different stereoisomers, salts or solvates of pinoxaden, sulfonylureas or safeners, as well as their amounts, can be combined to create different combinations. Thus, for example, a formulation of the invention may comprise
(i) between 0.2 and 15 w/w% of pinoxaden;
(ii) between 0.1 and 10 w/w % of mesosulfuron-methyl; and
(iii) 0.2 and 10 w/w% of an antioxidant, selected from the group consisting of butylated hydroxyanisole (BHA) and tert-butylhydroquinone (TBHQ), for example between 2 and 8 w/w%
   optionally, any other agrochemical acceptable additives and/or solvents;
   wherein said pinoxaden, stereoisomers, salts or solvates thereof and said sulfonylurea have improved stability when compared with the same composition without the antioxidant.

Alternatively, the formulation of the invention may comprise
(i) between 0.2 and 15 w/w% of pinoxaden;
(ii) between 0.1 and 10 w/w % of mesosulfuron or mesosulfuron-methyl;
(iii) between 0.01 and 10 w/w%, for example, between 2 and 8, for example between 3 and 7 w/w%, of an antioxidant selected from the group consisting of butylated hydroxyanisole (BHA) and tert-butylhydroquinone (TBHQ);
(iv) between 1 and 15 w/w % of a safener selected from the group consisting of cloquintocet, a salt of cloquintocet, cloquintocet-mexyl, mefenpyr, a salt of mefenpyr, mefenpyr-diethyl and mixtures thereof.

Alternatively, the formulation of the invention may comprise
(i) between 0.2 and 15 w/w% of pinoxaden;
(ii) between 0.1 and 10 w/w% of mesosulfuron-methyl;
(iii) between 0.5 and 7 w/w%, for example, between 1.5 and 7 w/w% or between 2 and 6.5 w/w%, of an antioxidant, selected from the group consisting of butylated hydroxyanisole (BHA) and tert-butylhydroquinone (TBHQ); and
(iv) between 1 and 15 w/w% of a safener

optionally, one or more agrochemical acceptable additive and/or carrier;
wherein said pinoxaden, stereoisomers, salts or solvates thereof and said sulfonylurea have improved stability when compared with the same composition without the antioxidant.

Alternatively, the formulation of the invention may consist of
(i) between 0.2 and 15 w/w% of pinoxaden, stereoisomers, salts or solvates thereof;
(ii) between 0.1 and 10 w/w % of a sulfonylurea;
(iii) between 0.01 and 10 w/w% of an antioxidant, selected from the group consisting of butylated hydroxyanisole (BHA) and tert-butylhydroquinone (TBHQ), preferably between 2 and 8 w/w%; and

optionally, one or more agrochemical acceptable additives and/or carrier;
wherein said pinoxaden, stereoisomers, salts or solvates thereof and said sulfonylurea have improved stability when compared with the same composition without the antioxidant selected from the group consisting of butylated hydroxyanisole (BHA) and tert-butylhydroquinone (TBHQ).

Alternatively, the formulation according to the invention consists of
(i) between 0.2 and 15 w/w% of pinoxaden;
(ii) between 0.1 and 10 w/w% of a sulfonylurea herbicide, for example, mesosulfuron-methyl;
(iii) between 0.01 and 10 w/w% of an antioxidant, selected from the group consisting of butylated hydroxyanisole (BHA) and tert-butylhydroquinone (TBHQ), preferably between 2 and 8 w/w%; and
optionally, one or more agrochemical acceptable additive and/or carrier;
wherein said pinoxaden, stereoisomers, salts or solvates thereof and said sulfonylurea have improved stability when compared with the same composition without the antioxidant.

### EXAMPLES

### Example 1: Formation of solids comprising pinoxaden and an antioxidant

Samples for compositional analysis were prepared by dissolving 7 w/w% BHA or TBHQ in canola oil. Then, at room temperature 5 w/w% of pinoxaden was added and stirred for 10 minutes. The solution was filtered, or solids allowed to settle followed by decantation of the liquid. The composition of the solid was analyzed and the results are summarized in Table 1.

**Table 1: compositional analysis of solids**

| **Sample No.** | **Sample** | **Pinoxaden (w/w %)** | **Antioxidant (w/w %)** | **Weight ratio Antiox.: PXD** | **Molar Ratio Antiox.:PXD** |
|---|---|---|---|---|---|
| **1** | Pinox. in oil | 65.69 | | | |
| **2** | Pinox. in oil + TBHQ | 32.56 | 11.25 | 0.35 | 0.83 |
| **3** | Pinox. in oil + BHA | 35.21 | 17.97 | 0.51 | 1.11 |

The results show that pinoxaden (PXD) and the antioxidants interact to form a solid.

### Example 2: Formation of crystals and XRD

Cocrystals were prepared by dissolving the antioxidant at 7wt% in canola oil at room temperature under stirring (300 rpm) until fully dissolved. Then at room temperature 5wt% of pinoxaden was added and stirred (300 rpm) for 24 hrs. The solution was filtered. Alternatively, the solution can be left to allow any solids to settle and then the liquid can be decanted.
In this way cocrystals of BHA with pinoxaden and TBHQ with pinoxaden were prepared and analyzed by X-ray powder diffraction (XRPD), the results being shown in Figures 1 and 2, respectively. The powder XRD diffractograms were obtained according to the following procedure:
A standard monocrystal XRD substrate/sample holder with a 2cm diameter well of 0.5mm depth was filled with the sample material. Phase analysis of six samples were performed by the X-ray powder diffraction (XRPD) method. The data were collected at room temperature on a Panalytical Empyrean powder diffractometer (Cu K_{α} radiation, α=1.54178 Å) equipped with an X'Celerator linear detector and operated at V=40 kV, I= 30 mA. Scans were run in a 2q range of 4-35□ with step equal to ~0.033°, scan speed ~0.106° sec.

### Example 3: Example of composition according to the invention

An OD formulation was prepared by grinding the solid materials and intimately mixing them with the oil carrier (canola oil). The mixture included pinoxaden, mesosulfuron, a safener (mefenpyr-diethyl) and butylated hydroxyanisole (BHA) in the amounts indicated in Table 2.

**Table 2**

| | **w/w %** |
|---|---|
| **Mesosulfuron (98%)** | 1.24 |
| **Pinoxaden (97%)** | 6.30 |
| **Mefenpyr** | 3.71 |
| **Aerosil R972** | 3.11 |
| **Alkamuls VO 2003** | 5.18 |
| **Antioxidant: (BHA)** | 5 |
| **Canola oil** | 75.43 |
| Sum | 100 |

### Example 4: Stability test using BHA

Different OD formulations were prepared by grinding the solid materials and intimately mixing them with the oil carrier (canola oil). The mixtures included pinoxaden, a sulfonylurea (e.g. mesosulfuron), a safener (mefenpyr-diethyl) and butylated hydroxyanisole (BHA) in different ratios. For both active ingredients and for the safener stability (chemical degradation) was tested, and compared to a reference formulation without antioxidant.

Samples with the composition listed in Table 3 where prepare following the procedure described above

**Table 3**

| | **A (comparative)** | **B** | **C** | **D** | **E** | **F** |
|---|---|---|---|---|---|---|
| | **w/w %** | **w/w %** | **w/w %** | **w/w %** | **w/w %** | **w/w %** |
| **Mesosulfuron (98%)** | 1.24 | 1.24 | 1.24 | 1.24 | 1.24 | 1.24 |
| **Pinoxaden (97%)** | 6.30 | 6.30 | 6.30 | 6.30 | 6.30 | 6.30 |
| **Mefenpyr** | 3.71 | 3.71 | 3.71 | 3.71 | 3.71 | 3.71 |
| **Rheological modifier** | 3.11 | 3.11 | 3.11 | 3.11 | 3.11 | 3.11 |
| **emulsifier** | 5.18 | 5.18 | 5.18 | 5.18 | 5.18 | 5.18 |
| **Antioxidant: (BHA)** | 0 | 1 | 2 | 3 | 4 | 5 |
| **Canola oil** | 80.43 | 79.43 | 78.43 | 77.43 | 76.43 | 75.43 |
| sum | 100 | 100 | 100 | 100 | 100 | 100 |

The samples of Table 3 were incubated for 2, a3 or 4 weeks at a temperature of 54°C. Then, the degradation of the two herbicides was measured and compared to the reference formulation (formulation A). The results for pinoxaden are shown in Table 4. The results for mesosulfuron are provided in Table 5. The first line of Table 4 and Table 5 provides the starting concentration in the formulation of the corresponding herbicide in w/w %. The second, third and fourth lines provide the concentration of the herbicide after 2, 3 and 4 weeks respectively, in w/w %, wherein the number in brackets reflects for each concentration value the corresponding % in which the concentration of the herbicide is reduced, with respect to the initial concentration.

**Table 4: w/w % of Pinoxaden in the samples (degradation from initial concentration)**

| | **A** | **B** | **C** | **D** | **E** | **F** |
|---|---|---|---|---|---|---|
| Start conc | 6.12 | 6.12 | 6.12 | 6.12 | 6.12 | 6.12 |
| 2 weeks @ 54°c | 5.58 (-8.82%) | 6.13 (0%) | 6.01 (-2%) | 6.07 (-0.8%) | 6.15 (0%) | 6.02 (-1.6%) |
| 3 weeks @ 54°c | 5.55 (-9.3%) | 5.74 (-6.2%) | 5.97 (-2.5%) | 6.00 (-1.9%) | 6.13 (0%) | 5.92% (-3.3) |
| 4 weeks @ 54°c | 5.33 (-12.9%) | | | | | 5.87 (-4.1%) |

**Table 5: w/w % of Mesosulfuron in the samples (degradation from initial concentration)**

| | **A** | **B** | **C** | **D** | **E** | **F** |
|---|---|---|---|---|---|---|
| Starting conc. | 1.21 | 1.21 | 1.21 | 1.21 | 1.21 | 1.21 |
| 2 weeks @ 54°c | 1.11 (-8.2%) | 1.15 (-4.9%) | 1.13 (-6.6%) | 1.12 (-7.4%) | 1.19 (-1.6%) | 1.20 (-0.8%) |
| 3 weeks @ 54°c | 1.08 (-10.7%) | 1.07 (-11.5%) | 1.14 (-5.8%) | 1.12 (-7.4%) | 1.20 (-0.8%) | 1.18% (-2.4) |
| 4 weeks @ 54°c | 1.03 14.8%) | | | | | 1.17 (-3.3%) |

### Example 5: Stability Test using TBHQ

Using the same procedure described in Examples 3 and 4, the formulation G described in Table 6 was prepared.

**Table 6**

| | **% w/w** |
|---|---|
| **Mesosulfuron (98%)** | 1.24 |
| **Pinoxaden (97%)** | 6.30 |
| **Mefenpyr** | 3.71 |
| **Silica (Rheological modifier)** | 3.11 |
| **Emulsifier (non-ionic)** | 5.18 |
| **tert-Butylhydroquinone (TBHQ)** | 3 |
| **Canola oil** | 77.43 |
| Sum | 100 |

The sample of Table 6 was incubated for 2 or 3 weeks at a temperature of 54°C. Then, the degradation of the two herbicides and of the safener was measured and compared with the reference formulation containing no antioxidant (formulation A). The results are provided in Table 7. The first line of Table 7 provides the starting concentration in the formulation of the corresponding herbicide or herbicide in w/w %. Below, the second and third lines provide the concentration of the herbicide after 2 and 3 weeks respectively, in w/w %. The fourth, fifth and sixth columns provide the corresponding % in which the concentration of the herbicide is reduced, with respect to the initial concentration. In all cases the value given in brackets corresponds to the result of the reference example (formulation A).

**Table 7**

| | **Starting Concentrations (w/w %)** | | | **Variation (%) in Concentration** | | |
|---|---|---|---|---|---|---|
| | **Mesosulf.** | **Pinox.** | **Mefen.** | **Mesosulf .** | **Pinox.** | **Mefen.** |
| | 1.21 | 6.36 | 3.68 | - | - | - |
| 2 weeks @ 54°c | 1.15 (1.11) | 6.20 (5.58) | 3.67 | -4.96 (-8.2) | -2.52 (-8.82) | -0.27 |
| 3 weeks @ 54°c | 1.16 (1.08) | 6.16 (5.55) | 3.68 | -4.13 (-10.7) | -3.14 (-9.3) | 0.00 |

From the results of Examples 3 and 4 it can be clearly observed that the presence of antioxidant, selected from the group consisting of butylated hydroxyanisole (BHA) and *tert-*butylhydroquinone (TBHQ), in the formulation has a stabilizing effect on the pinoxaden and on the sulfonylureas (mesosulfuron) co-formulated with pinoxaden, delaying their chemical degradation, or even avoiding it completely (see in formulation E, Table 4 how no degradation of pinoxaden was observed).

### Example 6: Stability Test of a mixture of Pinoxaden and Thiencarbazone-methyl

Using the same procedure described in Examples 3 and 4, the formulation H and the formulation reference H1 described in Table 8 were prepared.

**Table 8**

| | **H (% w/w)** | **Reference H1 (%w/w)** |
|---|---|---|
| **Thiencarbazone** | 0.85 | 0.85 |
| **Pinoxaden (99%)** | 10.20 | 10.20 |
| **Mefenpyr** | 5.15 | 5.15 |
| **Silica (Rheological modifier)** | 3.0 | 3.0 |
| **Emulsifier (non-ionic)** | 5.0 | 5.0 |
| **BHA** | 5.0 | 0 |
| **Canola oil** | 70.80 | 75.80 |
| Sum | 100 | 100 |

The samples of Table 8 were incubated for 2 or 3 weeks at a temperature of 54°C. Then, the degradation of the two herbicides and of the safener was measured and compared. The results are provided in Table 9. The first line of Table 9 provides the starting concentration in the formulation of the corresponding herbicide or herbicide in w/w %. Below, the second and third lines provide the concentration of the herbicide after 2 and 3 weeks respectively, in w/w %. The fourth, fifth and sixth columns provide the corresponding % in which the concentration of the herbicide is reduced, with respect to the initial concentration. In all cases the value given in brackets corresponds to the result of the reference example (formulation H1).

**Table 9**

| | **Starting Concentrations (w/w %)** | | | **Variation (%) in Concentration** | | |
|---|---|---|---|---|---|---|
| | **Thiencarb.** | **Pinox.** | **Mefen.** | **Thiencarb .** | **Pinox.** | **Mefen.** |
| | 0.71 (0.76) | 9.37 (9.35) | 4.61 (4.91) | - | - | - |
| 2 weeks @ 54°c | 0.72 (0.71) | 9.44 (9.70) | 4.63 (4.90) | -0.9 (-6.5) | -0.3 (-1.5) | -0.00 (-0.00) |
| 3 weeks @ 54°c | 0.70 (0.74) | 9.30 (9.71) | 4.62 (4.92) | -1.0 (-3.0) | -0.8 (-1.3) | -0.00 (-0.00) |

From the results it can be clearly observed that the presence of antioxidant, selected from the group consisting of butylated hydroxyanisole (BHA) and *tert*-butylhydroquinone (TBHQ), in the formulation has a stabilizing effect on the pinoxaden and on the sulfonylureas (thiencarbazone-methyl), delaying their chemical degradation.

## Claims

1. A co-crystal comprising pinoxaden, salts, solvates or stereoisomers thereof and an antioxidant, wherein the antioxidant is selected from the group consisting of butylated hydroxyanisole (BHA) and tert-butylhydroquinone (TBHQ).

2. The co-crystal according to claim 1 comprising pinoxaden, its stereoisomers, salts or solvates thereof and butylated hydroxyanisole (BHA).

3. The co-crystal according to claim 2, wherein the XRD powder diffraction pattern shows at least one peak expressed as a 2θ angle selected from the group consisting of 5.32, 13.09, 15.08, 18.60 and 23.15.

4. The co-crystal according to claim 1 comprising pinoxaden, its stereoisomers, salts or solvates thereof and tert-butylhydroquinone (TBHQ).

5. The co-crystal according to claim 4, wherein the XRD powder diffraction pattern shows at least one peak expressed as a 2θ angle selected from the group consisting of 5.64, 6.64, 7.95, 11.55, 13.18, 22.47, 23.11 and 24.55.

6. Process for the preparation of a co-crystal as defined in any of claims 1 to 5 comprising mixing pinoxaden, stereoisomers, salts or solvates thereof with an antioxidant which is selected from the group consisting of butylated hydroxyanisole (BHA) and *tert-*butylhydroquinone (TBHQ) in an organic solvent.

7. A herbicidal formulation comprising a co-crystal as defined in any of claims 1 to 5, and optionally one or more agrochemical acceptable additives and/or carriers.

8. The herbicidal formulation according to claim 7, comprising:
(i) between 0.2 and 15 w/w% of pinoxaden, stereoisomers, salts or solvates thereof;
(ii) between 0.1 and 10 w/w % of a sulfonylurea;
(iii) between 0.01 and 10 w/w% of an antioxidant, selected from the group consisting of butylated hydroxyanisole (BHA) and tert-butylhydroquinone (TBHQ);
and optionally, one or more agrochemical acceptable additive and/or carrier; wherein said pinoxaden, stereoisomers, salts or solvates thereof and said sulfonylurea have improved stability when compared with the same composition without the antioxidant.

9. The formulation according to any of claims 7 or 8, comprising:
(i) between 0.2 and 15 w/w% of pinoxaden;
(ii) between 0.1 and 10 w/w % of mesosulfuron or mesosulfuron-methyl;
(iii) between 0.01 and 10 w/w% of an antioxidant, selected from the group consisting of butylated hydroxyanisole (BHA) and ie/t-butylhydroquinone (TBHQ);
(iv) between 1 and 15 w/w % of a safener selected from the group consisting of cloquintocet, a salt of cloquintocet, cloquintocet-mexyl, mefenpyr, a salt of mefenpyr, mefenpyr- diethyl and mixtures thereof.

10. Use of the formulation defined in any of claims 7-9 for the control of undesired plants.

11. A method for the control of an undesired plant comprising contacting the formulation as defined in any of claims 7-9 with the locus of said undesired plant.

12. Use of a co-crystal comprising an antioxidant and a compound of formula (I), stereoisomers, salts or solvates thereof, to improve the chemical stability of said compound with respect to the stability said compound would have in the absence of the antioxidant;
wherein said compound of formula (I) is pinoxaden, stereoisomers, salts or solvates thereof;
and wherein the antioxidant is selected from the group consisting of butylated hydroxyanisole (BHA) and tert-butylhydroquinone (TBHQ).

13. A method for storing pinoxaden, stereoisomers, salts or solvates thereof, comprising the step of forming a co-crystal as defined in any of claims 1-5 by contacting with an antioxidant said pinoxaden, stereoisomers, salts or solvates thereof;
wherein the antioxidant is selected from the group consisting of butylated hydroxyanisole (BHA) and tert-butylhydroquinone (TBHQ).

14. A method of increasing the shelf life of pinoxaden, stereoisomers, salts or solvates thereof, comprising the step of forming a co-crystal as defined in any of claims 1-5 by contacting with an antioxidant said pinoxaden, stereoisomers, salts or solvates thereof;
wherein the antioxidant is selected from the group consisting of butylated hydroxyanisole (BHA) and tert-butylhydroquinone (TBHQ).

## Patentansprüche

1. Ein Co-Kristall umfassend Pinoxaden, Salze, Solvate oder Stereoisomere davon und ein Antioxidans, wobei das Antioxidans gewählt ist aus der Gruppe bestehend aus buthyliertem Hydroxianisol (BHA) und tert-Butylhydrochinon (TBHQ).

2. Das Co-Kristall gemäß Anspruch 1, umfassend Pinoxaden, seine Stereoisomere, Salze oder Solvate davon und buthyliertes Hydroxianisol (BHA).

3. Das Co-Kristall gemäß Anspruch 2, wobei das XRD-Pulverdiffraktionsmuster mindestens einen Peak zeigt, formuliert als ein 2θ-Winkel, gewählt aus der Gruppe bestehend aus 5.32, 13.09, 15.08, 18.60 und 23.15.

4. Das Co-Kristall gemäß Anspruch 1, umfassend Pinoxaden, seine Stereoisomere, Salze oder Solvate davon und tert-Butylhydrochinon (TBHQ).

5. Das Co-Kristall gemäß Anspruch 4, wobei das XRD-Pulverdiffraktionsmuster mindestens einen Peak zeigt, formuliert als ein 2θ-Winkel, gewählt aus der Gruppe bestehend aus 5.64, 6.64, 7.95, 11.55, 13.18, 22.47, 23.11 und 24.55.

6. Verfahren zur Herstellung eines Co-Kristalls, wie definiert in irgendeinem der Ansprüche 1 bis 5, umfassend das Mischen von Pinoxaden, Stereoisomeren, Salzen oder Solvaten davon mit einem Antioxidans, welches gewählt ist aus der Gruppe bestehend aus buthyliertem Hydroxianisol (BHA) und tert-Butylhydrochinon (TBHQ) in einem organischen Lösungsmittel.

7. Eine Herbizid-Formulierung, umfassend ein Co-Kristall, wie definiert in irgendeinem der Ansprüche 1 bis 5 und wahlweise ein oder mehrere agrochemisch verträgliche Zusatzstoffe und/oder Träger.

8. Die Herbizid-Formulierung gemäß Anspruch 7, umfassend:
(i) zwischen 0,2 und 15 Gew.-% von Pinoxaden, Stereoisomere, Salze oder Solvate davon;
(ii) zwischen 0,1 und 10 Gew.-% von einem Sulfonylharnstoff;
(iii) zwischen 0,01 und 10 Gew.-% von einem Antioxidans, gewählt aus der Gruppe bestehend aus buthyliertem Hydroxianisol (BHA) und tert-Butylhydrochinon (TBHQ);
und wahlweise ein oder mehrere agrochemisch verträgliche Zusatzstoffe und/oder Träger; wobei die Pinoxaden, Stereoisomere, Salze oder Solvate davon und der Sulfonylharnstoff eine verbesserte Stabilität aufweisen, im Vergleich zur gleichen Zusammensetzung ohne das Antioxidans.

9. Die Formulierung gemäß irgendeinem der Ansprüche 7 oder 8, umfassend:
(i) zwischen 0,2 und 15 Gew.-% von Pinoxaden;
(ii) zwischen 0,1 und 10 Gew.-% von Mesosulfuron oder Mesosulfuron-methyl;
(iii) zwischen 0,01 und 10 Gew.-% von einem Antioxidans, gewählt aus der Gruppe bestehend aus buthyliertem Hydroxianisol (BHA) und tert-Butylhydrochinon (TBHQ);
(iv) zwischen 1 und 15 Gew.-% von einem Safener, gewählt aus der Gruppe bestehend aus Cloquintocet, einem Salz von Cloquintocet, Cloquintocet-mexyl, Mefenpyr, einem Salz von Mefenpyr, Mefenpyr-diethyl und Mischungen davon.

10. Verwendung der Formulierung, definiert in irgendeinem der Ansprüche 7 bis 9, für die Bekämpfung unerwünschter Pflanzen.

11. Ein Verfahren zur Bekämpfung von einer unerwünschten Pflanze, umfassend das In-Kontakt-bringen der Formulierung, wie definiert in irgendeinem der Ansprüche 7 bis 9, mit dem Standort der unerwünschten Pflanze.

12. Verwendung von einem Co-Kristall, umfassend ein Antioxidans und eine Verbindung der Formel (I), Stereoisomere, Salze oder Solvate davon, um die chemische Stabilität der Verbindung im Vergleich zu der Stabilität der Verbindung in Abwesenheit von dem Antioxidans zu verbessern;
wobei die Verbindung der Formel (I) Pinoxaden, Stereoisomere, Salze oder Solvate davon ist;
und wobei das Antioxidans gewählt ist aus der Gruppe bestehend aus buthyliertem Hydroxianisol (BHA) und tert-Butylhydrochinon (TBHQ).

13. Ein Verfahren zur Lagerung von Pinoxaden, Stereoisomeren, Salzen oder Solvaten davon, umfassend den Schritt der Bildung eines Co-Kristalls, wie definiert in irgendeinem der Ansprüche 1-5, durch das In-Kontakt-bringen des Pinoxadens, der Stereoisomere, Salze oder Solvate davon mit einem Antioxidans;
wobei das Antioxidans gewählt ist aus der Gruppe bestehend aus buthyliertem Hydroxianisol (BHA) und tert-Butylhydrochinon (TBHQ).

14. Ein Verfahren zur Verlängerung der Haltbarkeit von Pinoxaden, Stereoisomeren, Salzen oder Solvaten davon, umfassend den Schritt der Bildung eines Co-Kristalls, wie definiert in irgendeinem der Ansprüche 1-5, durch In-Kontakt-bringen des Pinoxadens, der Stereoisomere, Salze oder Solvate davon mit einem Antioxidans;
wobei das Antioxidans gewählt ist aus der Gruppe bestehend aus buthyliertem Hydroxianisol (BHA) und tert-Butylhydrochinon (TBHQ).

## Revendications

1. Co-cristal comprenant du pinoxadène, ses sels, solvates ou stéréoisomères et un antioxydant, où l'antioxydant est choisi dans le groupe constitué par l'hydroxyanisole butylé (BHA) et le tert-butylhydroquinone (TBHQ).

2. Le co-cristal selon la revendication 1 comprenant du pinoxadène, ses stéréoisomères, ses sels ou solvates et de l'hydroxyanisole butylé (BHA).

3. Le co-cristal selon la revendication 2, où le diagramme de diffraction des rayons X sur poudre montre au moins un pic exprimé sous forme d'angle 20 choisi dans le groupe constitué par 5,32, 13,09, 15,08, 18,60 et 23,15.

4. Le co-cristal selon la revendication 1 comprenant du pinoxadène, ses stéréoisomères, ses sels ou solvates et de la tert-butylhydroquinone (TBHQ).

5. Le co-cristal selon la revendication 4, où le diagramme de diffraction des poudres XRD montre au moins un pic exprimé sous forme d'angle 20 choisi parmi le groupe constitué de 5,64, 6,64, 7,95, 11,55, 13,18, 22,47, 23,11 et 24,55.

6. Procédé de préparation d'un co-cristal tel que défini dans l'une quelconque des revendications 1 à 5, comprenant le mélange de pinoxadène, de ses stéréoisomères, sels ou solvates avec un antioxydant choisi dans le groupe constitué par l'hydroxyanisole butylé (BHA) et la tert-butylhydroquinone (TBHQ) dans un solvant organique.

7. Formulation herbicide comprenant un co-cristal tel que défini dans l'une quelconque des revendications 1 à 5, et éventuellement un ou plusieurs additifs et/ou excipients acceptables en agrochimie.

8. Formulation herbicide selon la revendication 7, comprenant :
(i) entre 0,2 et 15 % en poids de pinoxadène, de ses stéréoisomères, sels ou solvates ;
(ii) entre 0,1 et 10 % en poids d'une sulfonylurée ;
(iii) entre 0,01 et 10 % d'un antioxydant, choisi dans le groupe constitué par l'hydroxyanisole butylé (BHA) et la tert-butylhydroquinone (TBHQ) ;
et, éventuellement, un ou plusieurs additifs et/ou excipients acceptables en agrochimie ; où ledit pinoxadène, ses stéréoisomères, ses sels ou ses solvates et ladite sulfonylurée ont une stabilité améliorée par rapport à la même composition sans l'antioxydant.

9. La formulation selon l'une quelconque des revendications 7 ou 8, comprenant :
(i) entre 0,2 et 15 % en poids de pinoxadène ;
(ii) entre 0,1 et 10 % en poids de mésosulfuron ou de mésosulfiiron-méthyle ;
(iii) entre 0,01 et 10 % en poids d'un antioxydant, choisi dans le groupe constitué par
l'hydroxyanisole butylé (BHA) et la tert-butylhydroquinone (TBHQ) ;
(iv) entre 1 et 15 % en poids d'un phytoprotecteur choisi dans le groupe constitué par le cloquintocet, un sel de cloquintocet, le cloquintocet-mexyl, le méfenpyr, un sel de méfenpyr, le méfenpyr-diéthyl et leurs mélanges.

10. Utilisation de la formulation définie dans l'une quelconque des revendications 7 à 9 pour lutter contre les plantes indésirables.

11. Procédé pour lutter contre une plante indésirable, comprenant la mise en contact de la formulation telle que définie dans l'une quelconque des revendications 7 à 9 avec le locus de ladite plante indésirable.

12. Utilisation d'un co-cristal comprenant un antioxydant et un composé de formule (I), ses stéréoisomères, ses sels ou ses solvates, pour améliorer la stabilité chimique dudit composé par rapport à la stabilité que ledit composé aurait en l'absence de l'antioxydant ;
où ledit composé de formule (I) est le pinoxadène, ses stéréoisomères, ses sels ou ses solvates ;
et où l'antioxydant est choisi dans le groupe constitué par l'hydroxyanisole butylé (BHA) et la tert-butylhydroquinone (TBHQ) .

13. Procédé de stockage du pinoxadène, de ses stéréoisomères, sels ou solvates, comprenant l'étape consistant à former un co-cristal tel que défini dans l'une quelconque des revendications 1 à 5 en mettant en contact ledit pinoxadène, ses stéréoisomères, sels ou solvates avec un antioxydant ;
où l'antioxydant est choisi dans le groupe constitué par l'hydroxyanisole butylé (BHA) et la tert-butylhydroquinone (TBHQ) .

14. Procédé pour augmenter la durée de conservation du pinoxadène, de ses stéréoisomères, sels ou solvates,
comprenant l'étape consistant à former un co-cristal tel que défini dans l'une quelconque des revendications 1 à 5 en mettant en contact ledit pinoxadène, ses stéréoisomères, sels ou solvates avec un antioxydant ;
où l'antioxydant est choisi parmi le groupe constitué par l'hydroxyanisole butylé (BHA) et la tert-butylhydroquinone (TBHQ).
